(19) Europäisches Patentamt
European Patent Office
Office européen des brevets

(11) **EP 4 617 703 A1**

(12) **EUROPEAN PATENT APPLICATION**

(43) Date of publication:
**17.09.2025  Bulletin 2025/38**

(21) Application number: **24163711.5**

(22) Date of filing: **15.03.2024**

(51) International Patent Classification (IPC):
***G01R 33/565*** (2006.01)

(52) Cooperative Patent Classification (CPC):
**G01R 33/56509;** G01R 33/56563; G01R 33/5676

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Siemens Healthineers AG
91301 Forchheim (DE)**
• **The General Hospital Corporation
Boston, MA 02114 (US)**

(72) Inventors:
• **Polak, Daniel
91052 Erlangen (DE)**

• **Cauley, Stephen Farman
Winchester, 01890 (US)**
• **Deck, Jeanette
91052 Erlangen (DE)**
• **Liu, Wei
91052 Erlangen (DE)**
• **Splitthoff, Daniel Nicolas
91080 Uttenreuth (DE)**
• **Wald, Lawrence L.
Cambridge, MA, 02138 (US)**

(74) Representative: **Siemens Healthineers
Patent Attorneys
Postfach 22 16 34
80506 München (DE)**

(54) **METHOD FOR ACQUIRING A MAGNETIC RESONANCE IMAGE DATASET**

(57)    The invention relates to method for acquiring a magnetic resonance image dataset of a field-of-view (S) using a gradient-echo imaging protocol. The method comprises acquiring additional k-space lines (4) within a central region (16) of k-space at intervals throughout the imaging protocol, the additional k-space lines (4) being used for estimating motion parameters of the field-of-view. The imaging protocol has been amended by inserting additional gradient blips (26) after at least some of the RF excitations (22), such that at least one additional gradient echo (25) is generated, allowing the acquisition of at least one additional k-space (4) line during one echo time.

FIG 3

**Description**

The invention relates to a method for acquiring a magnetic resonance image dataset, to a magnetic resonance imaging apparatus and to a computer program.

Independent of the grammatical term usage, individuals with male, female or other gender identities are included within the term.

Patient motion is one of the most common and costly of artefacts in Magnetic Resonance Imaging (MRI) and can seriously degrade the diagnostic quality of magnetic resonance (MR) exams.

Fast imaging protocols reduce the impact of motion. For example, parallel imaging techniques, as summarised in J. Hamilton, D. Franson, and N. Seiberlich "Recent Advances in Parallel Imaging for MRI," Prog. Nucl. Magn. Reson. Spectrosc., vol. 101, pp. 71-95, 2017, exploit the properties of modern multi-channel coil arrays to separate aliased pixels in the image domain or to estimate missing k-space data, using knowledge of nearby acquired k-space points, in order to allow scan time reduction by sampling a smaller number of phase encoding lines in k-space.

Some magnetic resonance imaging (MRI) motion correction techniques involve measuring the motion by tracking devices or navigator acquisitions.

By contrast, retrospective motion correction techniques correct for motion artefacts after the data. By including motion parameters into the MR forward model, these techniques account for the patient's motion in the final image reconstruction and therefore reduce motion artefacts. In some techniques, the motion data can be derived from the acquired k-space data itself. For multi-shot acquisitions, the goal in retrospective motion correction techniques is to extract the *per* shot motion parameters and the motion-free image simultaneously. This can be accomplished by either minimizing an image quality metric, such as image entropy, or by minimizing the data consistency error of a parallel "imaging + motion" forward model, as described in L. Cordero-Grande, E. J. Hughes, J. Hutter, A. N. Price, and J. V Hajnal, "Three-dimensional motion corrected sensitivity encoding reconstruction for multi-shot multi-slice MRI: Application to neonatal brain imaging," Magn. Reson. Med., vol. 79, no. 3, pp. 1365-1376, 2018, L. Cordero-Grande, R. Teixeira, E. Hughes, J. Hutter, A. Price, Hajnal, "Sensitivity Encoding for Aligned Multishot Magnetic Resonance Reconstruction," IEEE Trans. Comput. Imaging, vol. 2, no. 3, pp. 266-280, 2016 and M. W. Haskell, S. F. Cauley, and L. L. Wald, "TArgeted Motion Estimation and Reduction (TAMER): Data consistency based motion mitigation for MRI using a reduced model joint optimization," IEEE Trans. Med. Imaging, vol. 37, no. 5, pp. 1253-1265, 2018. For the latter, the motion and image vector are jointly estimated via an inversion of the non-linear forward model. This corresponds to a large-scale non-linear optimization problem that is typically computationally very expensive. Previously proposed methods alternate between optimizing just the image or the motion parameters while assuming the other to be fixed (see L. Cordero-Grande in Magn. Reson. Med.), instead of updating all optimization variables at once. Nevertheless, repeated updates of the imaging voxels lead to excessive computation that prohibits its use in clinical settings.

When the "imaging + motion" model and the underlying imaging protocol also include parallel imaging techniques which make use of the complex sensitivity profiles of multi-channel coil arrays, such as SENSE (SENSitivity Encoding) or ASSET (Array coil Spatial Sensitivity Encoding), it is referred to as "SENSE + motion" model.

In *"Scout accelerated motion estimation and reduction (SAMER)"*, Magn. Reson. Med., vol. 87, pp. 163-178, 2022, https://doi.org/10.1002/mrm.28971, D. Polak, D. N. Splitthoff, B. Clifford, W.-C. Lo, S. Huang, J. Conklin, L. L. Wald, K. Setsompop and S. Cauley propose a technique that utilizes a single rapid scout scan to drastically reduce the computational cost of motion estimation. The scout image contains centre of k-space information which is compared against the k-space data of the actual MR acquisition for each shot, to derive the subject's motion. This corresponds to registration of the k-space data with the scout image in k-space. This strategy is used to completely avoid the alternating optimization of subject motion and image volume, which is otherwise required in navigator-, tracking-free retrospective motion correction techniques. In the SAMER-technique, a motion trajectory of the subject is first estimated, and the motion trajectory is then used in a motion-aware parallel image reconstruction, using e.g. a "SENSE + motion" forward model, to yield the motion-mitigated image. This reduces the computational cost by several orders of magnitude, when compared to established alternating optimization methods.

In D. Polak, J. Hossbach, D. N. Splitthoff et al. "Motion guidance lines for robust data consistency-based retrospective motion correction in 2D and 3D MRI", Magn. Reson. Med. 2023:1-14, doi: 10.1002/mrm.29534, D. Polak et al. have extended the SAMER method to include the repeated acquisition of a small number of motion guidance lines in each shot, which are used for motion estimation by being compared with the data from the scout scan. This allows for very rapid and fully separable estimation of motion parameters shot-by-shot.

However, the SAMER method using additional motion guidance lines has the disadvantage that the additional motion guidance lines require additional scan time. In multi shot sequences (e.g. MPRAGE) the repetition time TR (e.g. ~2sec) is typically much larger than the echo train duration (~ 1sec), therefore playing additional guidance lines during the gap time of each TR does not increase the overall scan. By contrast, steady state sequences like T2* GRE constantly acquire data and hence there is no gap time which one can exploit for the guidance line acquisition. Long TEs further excacerbate the time penalty of the guidance lines.

It is therefore an object of the present invention to overcome the problems of the SAMER method, in particular the problem of prolonging the acquisition times of the imaging protocol. It is a further object of the invention to provide a method for acquiring MR image datasets for retrospective motion correction, which provides additional motion information.

These objects are met by a method for acquiring a magnetic resonance image dataset of a field-of-view including a body part of a subject, using a gradient-echo imaging protocol, in which spatial encoding is performed using phase encoding gradients along at least one phase encoding direction, and frequency encoding gradients along a frequency encoding direction, wherein k-space is sampled during the imaging protocol by using RF excitations followed by an echo time resulting in a gradient echo, wherein during each gradient echo, an imaging k-space line oriented along the frequency encoding direction is sampled. The method comprises acquiring additional k-space lines within a central region of k-space at intervals throughout the imaging protocol, the additional k-space lines being used for estimating motion parameters of the field-of-view. The gradient-echo imaging protocol has been amended by inserting additional gradient blips after at least some of the RF excitations, such that at least one additional gradient echo is generated, allowing the acquisition of at least one additional k-space line during one echo time.

The additional k-space lines are herein also referred to as "motion guidance lines" or "guidance lines". "Additional" means that the k-space lines are acquired in addition to those that are required under the imaging protocol to acquire the MR image, i.e. they are redundant when it comes to image reconstruction.

Gradient echo (GRE) magnetic resonance imaging is a common imaging technique used for in many diagnostic settings. The invention exploits the dead-time between the RF excitation and the data acquisition, or between data acquisition and the next RF excitation, by inserting an additional gradient echo after the RF excitation, for example at an echo time of 2 to 20ms, preferably 3 to 10ms, e.g. 4 ms. This does not increase scan time or change the contrast of the imaging data. Additional gradient encoding blips are therefore inserted in order to allow the additional k-space line and an imaging k-space line to be collected from different k-space locations.

Depending on the length of the echo time, the additional gradient echo may be acquired between the RF excitation and the acquisition of the imaging data. Alternatively, in particular in gradient echo sequences with small/very small TE, the guidance lines may also be placed after the imaging echo. In other words, in this alternative, the additional gradient echo is acquired after the acquisition of the imaging data.

The gradient echo sequence is preferably a steady-state sequence. It is preferably not a multi shot sequence (e.g. MPRAGE), in which the repetition time TR is much larger than the echo train duration.

Susceptibility weighted imaging (SWI) is a variant of GRE using long TEs. SWI is part of routine clinical brain exams but also plays an important role in emerging specialized screening protocols, such as for the detection of ARIA (amyloid related imaging abnormalities) in Alzheimer's disease patients. However, the long echo times needed to produce T2*/SWI contrast cause long acquisition times and thus increase the susceptibility to patient motion, possibly leading to significant degradation in image quality. Therefore, the insertion of additional k-space lines has so far not been considered, since it will further increase the acquisition times. However, gradient-echo susceptibility-weighted (GRE-SWI) MR images are typically acquired with a long TE to ensure sufficient T2* contrast. The invention exploits the dead time between the RF excitation and the data acquisition, or between data acquisition and the next RF excitation, for inserting an additional gradient echo.

The imaging protocol is a susceptibility-weighted gradient-echo imaging protocol. SWI-MRI uses a type of MRI sequence that exploits the magnetic susceptibility differences of various tissues and their compounds, including de-oxygenated blood, iron and calcium, to enable contrast in MRI. SWI is particularly useful in investigating trauma and acute neurologic occurrences such as strokes in the brain, but may also be used to diagnose low-flow vascular malformations, cerebral micro-bleeds and brain tumour. The SWI imaging protocol may for example has a long echo time (TE) of 10ms to 100ms (at low field strengths of e.g. 0,5T), preferably 15ms to 50ms, more preferred 18ms to 25ms, in particular around 20ms - 30ms (at 3 Tesla). It is preferably a gradient echo sequence. The repetition time (TR) may for example be 5ms to 15ms, preferably 8ms to 12ms longer than the TE. The flip angle is preferably between 5° and 25°, more preferred between 10° and 20°. The imaging protocol is preferably flow-compensated. The main magnetic field strength may be any commercially used field strength, for example between 0.5 T and 7T. The invention is preferably applied to 3D GRE-SWI imaging protocols.

The thereby acquired susceptibility-weighted magnetic resonance image dataset, also referred to as "image dataset", or "image", is designed to be reconstructed using retrospective motion correction techniques, in particular the technique disclosed by D. Polak et al. in Magn. Reson. Med., vol. 87, pp. 163-178, 2022 and Magn. Reson. Med. 2023:1-14, doi: 10.1002/mrm.29534. The image dataset is typically acquired for diagnostic purposes and thus preferably has a high spatial resolution of, for example, an in-plane resolution of 0.3mm - 3mm, preferably 0.4-2mm. The voxel size may be for example e.g. 0,5 to 12mm$^3$, preferably 2 to 8mm$^3$. For a 2D image, the in-plane resolution may for example be 0.3mm - 2mm, preferably 0.4-1.2mm.

The imaging protocol may be a two-dimensional (2D) or three-dimensional (3D) imaging protocol. In a 2D-imaging protocol, a slice of the object may be selected during RF excitation using a slice-select gradient. During each shot, the k-space plane is sampled by using a phase encode gradient along one dimension and a frequency encoding gradient along another spatial dimension. In a 3D-imaging protocol, a larger volume of tissue is excited together. Spatial encoding is performed using phase-encoding gradients along two spatial dimensions, a partition direction and a phase-encode direction, and frequency encoding along the third dimension, referred to as readout direction. Accordingly, with each MR echo, a k-space line oriented along the readout direction is sampled, at varying partition (PAR) and phase-encode (PE) positions.

By modifying the phase-encoding gradient before each readout, it is possible to design different sampling orders (also referred to as "reordering", "reordering scheme", "sampling scheme" or "reordering pattern"), which may refer to the temporal order in which the k-space lines are acquired. For example, in a linear sampling scheme, the phase-encoding gradient(s) is/are increased incrementally or step-by-step for each k-space line, resulting in a sampling of k-space line-by-line, from one end of k-space to the other. The data acquisition in 3D GRE-SWI usually employs a linear reordering scheme, i.e., it iterates over all partition encoding lines (inner loop), before continuing with the next phase encoding position (outer loop).

According to a preferred embodiment, the method comprises acquiring a low-resolution scout image data set of the field-of-view. Thereby, the scout can serve as a reference when estimating the motion states from the motion guidance lines.

The scout is acquired so fast that it can be assumed to represent a single motion state (i.e. the subject has not moved during the scout acquisition). The low-resolution scout image preferably covers the same field-of-view as the magnetic resonance image dataset. Thus, in case of a 3D image, it covers the same volume. In case the image dataset is a stack of 2D slices, the scout image comprises also a stack of low-resolution 2D slices. However, it is possible that the low-resolution scout comprises fewer slices than the high-resolution image, for example only every second or third slice. Similarly it is possible that the scout covers a larger volume, for a better stability in the motion detection step. The low-resolution scout image may have a spatial resolution of 2-8mm, preferably 3-5mm, for example 4mm, in the phase-encoding direction(s). Preferably, the acquisition of the scout is very rapid, requiring e.g. 1-5 sec, preferably 1-2 secs. It is preferably acquired once before or after the imaging protocol, preferably before. Thus, the acquisition of the low-resolution scout is preferably the first method step, though it may also be the last and may, in principle, also be performed in the middle of the imaging protocol. In a further step, the imaging protocol is carried out. It is advantageous for the scout image to be contrast matched to motion guidance lines, i.e. using the same TE and TR. Thereby the motion states can be accurately estimated.

Before executing the method of the invention, an MR examination will often comprise some preparatory scans. In particular, SENSE+motion reconstructions require a coil sensitivity map which is typically computed from a separate, very rapid reference scan. This scan is often the first scan of the acquisition, followed by the low-resolution scout and the imaging protocol according to an embodiment of the invention.

The additional k-space lines are disposed in the central region of k-space. Preferably, the central region is equivalent to a magnetic resonance image of low resolution, such as the scout image. In particular, the central region of k-space may be equivalent to a low-resolution image having a pixel/voxel size of $\geq 3$ mm, preferably $\geq 4$ mm, for example 3-7 mm, preferably 4-6 mm. It has been demonstrated to be advantageous for the guidance lines to be chosen close to the k-space centre, since low-frequency k-space information typically better captures the effect from patient motion than information from the k-space periphery. Moreover, in case the SAMER technique (Magn. Reson. Med., pp. 163-178, 2022) is used, the calibration samples should be chosen such that there is sufficient overlap between the scout and the calibration data for detecting motion, in particular sufficient common frequency overlap.

The inventive method may be executed on any medical or other MRI apparatus. The field-of-view includes a body part of a subject, which may be human or animal, in particular a patient to be examined. The image dataset is in particular acquired from a part of the body that is subject to undesired motion, for example the head or neck, a limb such as a leg, arm, knee, hand, or a part subjected to breathing motion such as the thorax or abdomen.

The additional k-space lines are used for estimating motion parameters of the field-of-view. The motion parameters are in particular a so-called motion trajectory, i.e. the motion parameters describe the position of the body part of the subject within the field-of-view at various time points throughout the imaging protocol, wherein the time points are separated by intervals. The method of the invention allows acquiring a motion guidance line (additional k-space line) every TR, which may for example be every 10-100ms, preferably every 20-50ms, i.e. typical repetition times (TR) for a SWI imaging protocol at 3T. In preferred embodiments, a motion state is not estimated from a single motion guidance line, but from a set of 2-16, preferably 4-8 motion guidance lines sampling different positions within the central region of k-space. However, even when using a set of motion guidance lines of each motion state, the temporal resolution which is attainable by using the dead-time before or after the imaging echo according to the invention, is more than enough for the purpose of motion trajectory estimation. Therefore, in some embodiments of the invention, additional k-space lines are not acquired during each repetition time, but only in a portion thereof, for example in 5-60 %, preferably 10-40% of the repetition times. This reduces the patient exposure to imaging gradients, thereby reducing the gradient switching noises and associated energy consumption for the imaging protocol. In other embodiments, additional k-space lines are acquired at each repetition time and are used for deducing further information, for example to estimate the BO-field inhomogeneities, to acquire an or another low-resolution image data set of the field-of-view, or to increase the time resolution of the estimated motion parameters.

According to an embodiment, the additional k-space lines are acquired from a set of 2 to 16, preferably 4 to 8, k-space positions within a central region of k-space, wherein each set of 2 to 16, preferably 4 to 8, additional k-space lines is used to estimate one motion state of the field-of-view, preferably a rigid-motion state. A rigid motion state can for example be described by a number of parameters, in particular three translation parameters and three rotation parameters. Each set of additional k-space lines typically comprises fewer k-space lines than would be required to reconstruct a scout image. The set of additional k-space lines is preferably acquired repeatedly during the imaging protocol, namely at - at least approximately - regular intervals. "At least approximately", when used in this application, may mean within ±15%, preferably within ±10%, most preferred within ±5%. Each set of additional k-space lines is intended to provide information on the position of the subject at the point in time at which the set was acquired, also referred to as motion state. Therefore, the interval at which the sets of additional k-space lines are acquired is preferably between 0.3s and 3.0s, more preferred between 0.5s and 2.0s, most preferred between 1.0s and 2.0s. By acquiring sets of guidance lines in more TRs, a motion state may even be estimated every 0.2-0.8 seconds. The sets of additional k-space lines are typically at the same position in k-space at each acquisition, but this is not mandatory.

The density of the additional k-space lines can vary during the acquisition, e.g. a) in order to be able to determine motion parameters with a higher temporal resolution for important k-space parts more (e.g. k-space centre) or b) in case the already detected motion parameters during the acquisition indicate a higher temporal resolution is required.

Each set of additional k-space lines (motion guidance lines) is preferably being acquired from consecutive RF excitations. Thereby, it is assured that each set represents one motion state of the subject, by minimizing the time delay between the acquisition of the additional k-space lines which are used to estimate one motion state.

According to an embodiment, the susceptibility-weighted gradient-echo imaging protocol is a 3D imaging protocol using a linear sampling order, wherein one set of 2-16, preferably 4-8 additional k-space lines is acquired during each iteration of an inner phase-encoding loop of the imaging protocol. The inner phase-encoding loop may for example be the partition encoding, in case of imaging protocols which iterate over all partition encoding lines in an inner loop, before continuing with the next phase encoding position in an outer loop. In order to achieve the preferred temporal resolution of 1-2 seconds, it is preferred to collect one set of, for example, four motion guidance lines during every run of the inner loop.

According to an embodiment, at least one additional k-space line is acquired during each repetition time, i.e. after each RF excitation, in particular if the echo time of the imaging protocol is long enough to accommodate at least one, possibly even two or three k-space lines, as for example in SWI imaging. Thus, embodiments in which (a) at least one additional k-space line is acquired after each RF excitation, and/or (b) two or three additional k-space lines are acquired after at least some RF excitations, are conceivable. These additional k-space lines may be used for a higher temporal resolution of the motion estimation.. More preferred, the additional k-space lines may be used to provide additional input, for example to estimate B0 field variations. This is because every guidance line echo acquired per excitation is acquired at a slightly different echo time, but at the same k-space location, and thus the change in signal across the echoes reflects the B0 inhomogeneity. Advantageously, this data is acquired from multiple k-space locations to get enough spatial encoding. According to an embodiment, of two separate low resolution images are acquired - using the additional k-space lines - at different TEs for generating a B0 field map.

Furthermore, the acquisition of the motion guidance lines can be interleaved with low-resolution images or navigators for additional pose estimation, or for estimating field homogeneities associated with the motion. In other words, only some of the additional k-space lines acquired per RF excitation are used as motion guidance lines to estimate motion states. Other additional k-space lines are used to acquire a low-resolution image or navigator. Furthermore, they can be used to estimate homogeneities in the BO-field which are also caused by motion and which may then be compensated for.

According to an embodiment, the phase of at least some of the additional k-space lines is used to estimate the BO-field inhomogeneities caused by motion. This may, for example, be done using the approach described by Y. Brackenier in "Data-driven motion-corrected brain MRI incorporating pose-dependent B0-fields", Magn. Reson. Med., vol. 88, no. 2, pp. 817-831, 2022. Therein, the data-driven retrospective motion correction includes modelling of pose-dependent changes in the BO-field in the head. The use of a physics-based BO-field model constrains the number of unknowns to be found, enabling motion correction based on data-consistency. Thus, the B0-fields may be estimated based on data-consistency by amending the SENSE+motion model by including an additional phase term $e^{in}$ into the forward model which may vary per motion state. Alternatively, the discrete Fourier operator may be replaced with a field-dependent Fourier operator.

According to an embodiment, the imaging protocol includes flow compensation gradients inserted between the acquisitions of the imaging k-space lines and the additional k-space lines, in order to reduce flow artefacts in the magnetic resonance image data set. Flow compensation gradients are known in the art. However, flow suppression is only required for the imaging echo, since the motion guidance lines are used for motion trajectory estimation only. Therefore, the flow compensation gradients should be inserted as close as possible before the acquisition of the imaging k-space lines, i.e. the data used for image reconstruction. This position is beneficial, since it reduces the required peak gradient strength and potential nerve stimulation. Preferably, the flow compensation gradients are designed to achieve full flow suppression in the imaging echo (imaging k-space lines), taking into account all guidance lines and imaging gradients.

According to a further aspect, the invention is also directed to a method for generating a motion-corrected magnetic resonance image dataset of an object, comprising receiving k-space data acquired using the acquisition method according to an embodiment of the invention, and receiving a low-resolution scout image dataset and sets of additional k-space lines acquired using the acquisition method according to an embodiment of the invention.

The motion correction algorithm comprises, in a first step, estimating motion parameters for each set of additional k-space lines by minimizing the data consistency error between the additional k-space lines and a forward model using the low-resolution scout scan as an estimate for the image dataset, wherein the forward model is described by an encoding matrix including the motion parameters to be estimated, Fourier encoding, and optionally subsampling and/or coil sensitivities of a multi-channel coil array. In a second step, the motion correction algorithm comprises estimating the motion-corrected image dataset by minimizing the data consistency error between the k-space data acquired in the imaging protocol and a forward model described by an encoding matrix, wherein the encoding matrix includes the motion parameters estimated in the first step for each set of additional k-space lines, Fourier encoding, and optionally subsampling and/or coil sensitivities of a multi-channel coil array.

The motion parameters may be rigid-body motion parameters, comprising e.g. three translational and three rotational parameters, or non-rigid motion parameters.

Thus, the minimisation problem is carried out in two steps: in a first step, motion parameters are estimated for each set of guidance lines by minimizing the data consistency error of the forward model, which may amount to a comparison with the low-resolution scout image. In a second step, the motion-corrected image is estimated using the motion parameters estimated in the first step. If motion dependent B0 fields were estimated, as explained above, this information may also be included into the image reconstruction / image estimation. Thereby, alternating repeated updates of the otherwise coupled optimisation variables x (image vector) and θ (motion parameters) is avoided. Rather, the rapid scout image dataset is used as an image estimate $\bar{x}$. This leads to a highly efficient optimisation problem that is fully separable across the echo trains and does not require repeated updates of x, which may comprise millions of imaging voxels. The minimisation problem may be derived from a SENSE parallel imaging forward model, as described in K. P. Pruessmann, M. Weiger, M. B. Scheidegger, and P. Boesiger, "SENSE: sensitivity encoding for fast MRI," Magn. Reson. Med., vol. 42, no. 5, pp. 952-962, 1999, with rigid body motion parameters included ("SENSE + motion" model). The overall method is described in Magn. Reson. Med. 2023:1-14, doi: 10.1002 / mrm.29534, which is incorporated herein by reference.

In a further aspect of the invention, a magnetic resonance imaging apparatus is provided which comprises a radio frequency controller configured to drive an RF-coil, preferably comprising a multi-channel coil-array, a gradient controller configured to control gradient coils, and a control unit configured to control the radio frequency controller and the gradient controller to execute the imaging protocol according to the invention. The MRI-apparatus may be a commercially available MRI-apparatus which has been programmed to perform the method of the invention.

According to a further aspect of the invention, a computer configured to generate a motion-corrected magnetic resonance image dataset is provided. The computer may be any computer comprising a sufficiently powerful processing unit, which may be a CPU or GPU, or several such processing units. Accordingly, the computer may be a PC, a server, a console of an MRI apparatus, but it also may be a computer that is remote from the MRI apparatus, it may be connected with it through the internet. Accordingly, the computer may also be a cloud computer, a remote server etc. The computer may also be a mobile device, such as a laptop, tablet computer or mobile phone.

According to a further aspect of the invention, a computer program is provided which includes program code, which causes a magnetic resonance imaging apparatus - such as the apparatus described herein - to execute the inventive method, in particular the inventive method for acquiring an MR image dataset. However, the program code may also encode the described method for generating a motion-corrected magnetic resonance image dataset, and the program code may run on a computer as described herein.

According to a further aspect, the invention is directed to a non-transitory computer-readable medium containing a computer program as described herein. The computer-readable medium may be any digital storage medium, such as a hard disc, a cloud, an optical medium such as a CD or DVD, a memory card such as a compact flash, memory stick, a USB-stick, multimedia stick, secure digital memory card (SD) etc.

All features disclosed with regard to the acquisition method may be combined with all features of method for generating a motion-corrected MRI dataset and vice versa. Also, all features of the disclosed methods may be embodied in the MRI apparatus, computer program and computer-readable storage medium according to other aspects of the invention and vice versa.

The accompanying drawings illustrate various example embodiments of various aspects of the invention. In the drawings:

Fig. 1     is a schematic representation of an MRI apparatus according to an embodiment of the invention;

Fig. 2     is a schematic representation of a three-dimensional k-space;

Fig. 3     is a sequence diagram of a SWI sequence with motion guidance lines;

Fig. 4     is an example image of a k-space illustrating the temporal acquisition order of the imaging k-space lines;

Fig. 5     is an illustration of an alternating optimization algorithm; and

Fig. 6     is an illustration of a SAMER optimization.

Similar elements are designated with the same reference signs in the drawings.

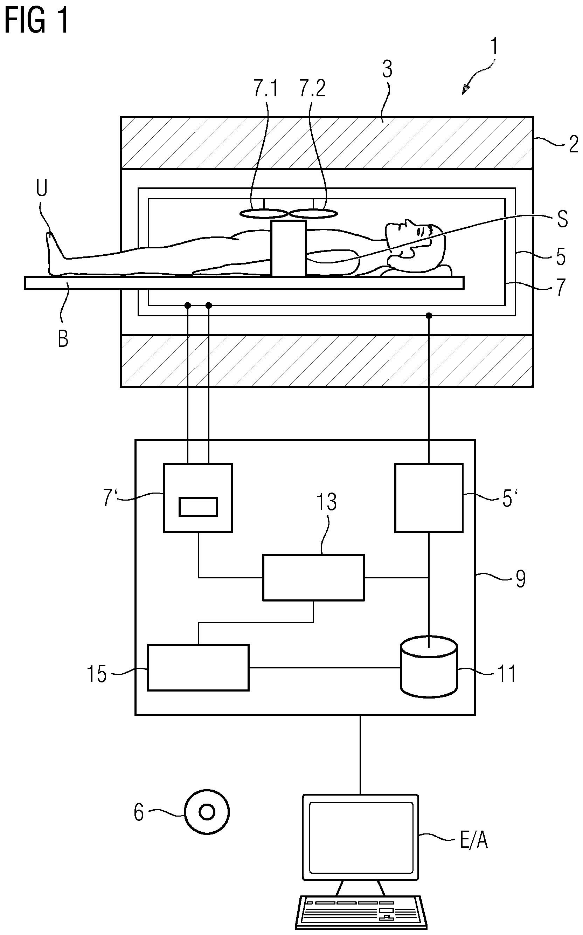

Fig. 1 schematically shows an inventive magnetic resonance (MR) apparatus 1. The MR apparatus 1 has an MR data acquisition scanner 2 with a main magnet 3 that generates the constant magnetic field $B_0$, a gradient coil arrangement 5 that generates the gradient fields, one or several radiofrequency (RF) antennas 7 for radiating and receiving RF signals, and a control computer 9 configured to perform the inventive method. The radio-frequency antennas 7 may include a multi-channel coil array comprising at least two coils, for example the schematically shown coils 7.1 and 7.2, which may be configured to transmit and/or receive RF signals (MR signals).

In order to acquire MR data from an examination subject U, for example a patient or a phantom, the subject U is introduced on a bed B into the sensitive volume or bore of the scanner 2. A 3D MR image can be acquired using a method according to an embodiment of the invention from a 3D volume S. The control computer 9 controls the MR apparatus 1, and the gradient coil arrangement 5 with a gradient controller 5' and the RF antenna 7 with a RF transmit/receive controller 7'. The RF antenna 7 has multiple channels corresponding to the multiple coils 7.1, 7.2 of the coil arrays, in which signals can be transmitted or received. The control computer 9 also has an imaging protocol processor 15 that determines the imaging protocol, including the acquisition of imaging and additional k-space lines. A control unit 13 of the control computer 9 is configured to execute all the controls and computation operations required for acquisitions. Intermediate results and final results required for this purpose or determined in the process can be stored in a memory 11 of the control computer 9. A user can enter control commands and/or view displayed results, for example image data, an input/output interface E/A. A non-transitory data storage medium 6 can be loaded into the control computer 9 and may be encoded with programming instructions (program code) that cause the control computer 9, and the various functional units thereof described above, to implement any or all embodiments of the method according to the invention.

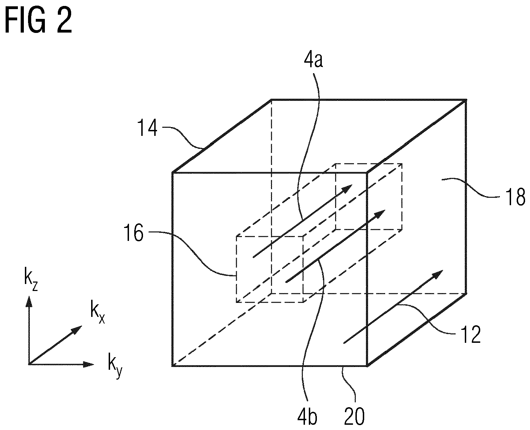

Fig. 2 illustrates a three-dimensional k-space 14 having dimension $k_x$ in frequency encoding (readout) direction, and $k_y$ and $k_z$ in the phase encode plane 20. The k-space volume 14 includes a central region 16 and a periphery 18. An imaging k-space line 12 acquired during one echo is illustrated in the periphery. On the other hand, additional k-space lines 4a, 4b as well as the k-space lines acquired for the low-resolution scout are located in the central region 16. In the phase encode plane 20, the central region 16 may cover about 1/12 to 1/16 in each direction, i.e. less than 1/100 of the total square phase encode plane 20.

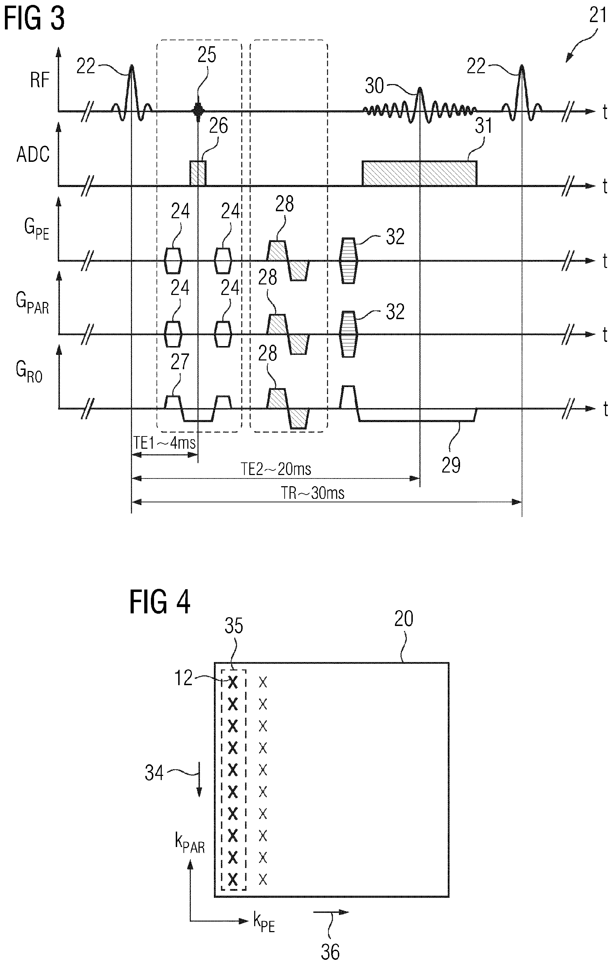

Fig. 3 illustrates a T2*-weighted imaging protocol 21 including the acquisition of motion guidance lines 4 according to an embodiment of the invention.

The diagram depicts the RF-pulses and signals on the top line, the ADC (analogue-to-digital converter) on the line below. This indicates when data is acquired. Below that are two lines for the two phase encoding gradients, wherein $G_{PE}$ indicates the phase encoding direction and $G_{PAR}$ the partition direction. The frequency encoding (readout) gradients are shown below. The diagram 21 illustrates one repetition time TR, which in this case is 30ms long. At the beginning of each TR, an RF excitation pulse 22 is transmitted. This is followed by gradient blips 24, which are used to direct the signal to one of the central positions in the phase encode plane 20, in which the motion guidance lines are acquired. The acquisition of an additional k-space line 4 in the shape of a guidance line echo 25 is indicated by the ADC at 26, and by the readout gradient 27. In the implemented example, the echo time TE1 of the guidance line echo, 25, was 4ms. Flow compensation gradients are played just before the acquisition of the imaging echo 30. Note that flow compensation gradients along the frequency encoding (readout) direction may also be played out, but are not shown in this diagram. Thus, after the flow compensation gradients 28, phase encode gradient blips 32 are played out in order to adjust the position of the imaging k-space lines 12 in the phase-encode plane 20, before the ADC 31 is switched on for the acquisition of the imaging echo 30 during readout gradient 29 at an echo time TE2 of 20ms in this example. 10ms after the last echo, the TR is repeated with RF pulse 22.

Fig. 4 illustrates the linear sequence ordering in GRE-SWI, wherein the acquisition of the imaging k-space lines 12 iterates through all partition encoding steps in an inner loop, indicated by arrow 34, before moving to the next phase encoding position, i.e. the next line along the outer loop direction 36. In an example, four additional k-space lines (guidance line echoes) are collected during each run of the inner loop, which is illustrated by the box 35. This enables motion trajectory estimation roughly once per second.

The sequence illustrated in Fig. 3 has been implemented and has been shown to yield improved image sharpness and vessel depiction in situations with step motion and breathing motion. Further, this SWI sequence with guidance lines and flow compensation according to an embodiment of the invention has achieved excellent image quality and flow artefact suppression, in addition to motion compensation.

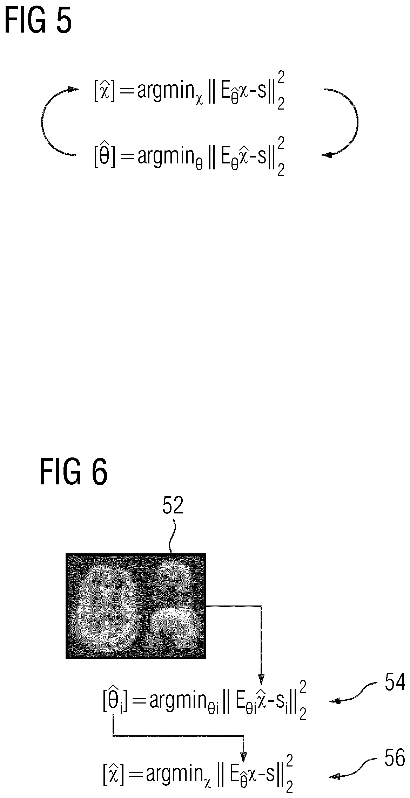

A retrospective motion correction technique which may be used on the MR signals acquired with the inventive acquisition method, and especially to estimate the motion states, will now be described with reference to Fig. 5 and 6. The mathematical model used is an extension of SENSE parallel imaging, as described in the above-cited paper by K. P. Pruessmann et al., with rigid-body motion parameters included into the forward model. The forward model or encoding operator $E_\theta$ for a given patient motion vector $\theta$ (comprising motion parameters over time) relates the motion-free image $x$ to the acquired multi-channel k-space data $s$. At each time point $i$ that is considered, e.g. the acquisition time of the sets of guidance lines, the subject's position is described by a new set of six rigid-body motion parameters $\theta_i$ that describe the 3D position of the object. Accordingly, the multi-channel k-space data $s_i$ acquired at time point $i$ may be related to the 3D image volume $x$ through image rotations $R_i$, image translations $T_i$, coil sensitivity maps $C$, Fourier operator $F$ and undersampling mask $M_i$ by the following formula 1

$$s_i = E_{\theta_i} x = M_i\, F\, C\, T_{\theta_i} R_{\theta_i} x \qquad [1]$$

In prior art methods, as illustrated in Fig. 5, both the motion corrected image vector $x$ and the motion vector (trajectory) $\theta$ are estimated by performing an alternating, repeated optimization between the image vector (formula 2) and the motion vector (formula 3):

$$[\hat{x}] = \mathrm{argmin}_x \|E_{\hat{\theta}} x - s\|_2 \qquad [2]$$

$$[\hat{\theta}] = \mathrm{argmin}_\theta \|E_\theta \hat{x} - s\|_2 \qquad [3]$$

This can lead to convergence issues as updates of $x$ and $\theta$ will be computed on inaccurate information. Moreover, the reconstruction is computationally demanding as repeated updates of $x$ (millions of imaging voxels) are needed.

Using one or several ultra-fast low-resolution scout scans 52, the motion trajectory may be directly estimated, as illustrated in Fig. 6, thus avoiding the time-consuming alternate optimization. The scout image 52, designated by $\tilde{x}$, approximates the motion-free image volume $\tilde{x}$ and each motion state can be determined independently by minimizing the data consistency error of the forward model 54:

$$[\hat{\theta}_i] = \mathrm{argmin}_{\theta_i} \|E_{\theta_i} \tilde{x} - s_i\|_2 \qquad [4]$$

In the method of the invention, the guidance lines are used instead of or in addition to the k-space data $s$ in this first step of the optimization. Preferably, only the guidance lines are used.

For the final image reconstruction, the motion states/parameters from each set of guidance lines are combined and the motion-mitigated image is obtained from solving a standard least-squares problem 56:

$$[\hat{x}] = \mathrm{argmin}_x \|E_{\hat{\theta}} x - s\|_2 \qquad [5]$$

This strategy completely avoids the difficult non-linear and non-convex joint optimization that contains millions of unknowns, as it only considers six rigid body parameters per motion optimization, and it does not require computationally costly full or partial updates to the image.

## Claims

1. A method for acquiring a magnetic resonance image dataset of a field-of-view (S) including a body part of a subject (U),

   using a gradient-echo imaging protocol (21), in which spatial encoding is performed using phase encoding gradients ($G_{PE}$, $GP_{AR}$) along at least one phase encoding direction ($k_y$, $k_z$), and frequency encoding gradients along a frequency encoding direction ($k_x$),
   wherein k-space (14,) is sampled during the imaging protocol (21) by using RF excitations (22) followed by an echo time resulting in a gradient echo (30), wherein during each gradient echo, an imaging k-space line (12) oriented along the frequency encoding direction is sampled,

wherein the method comprises acquiring additional k-space lines (4) within a central region (16) of k-space at intervals throughout the imaging protocol, the additional k-space lines (4) being used for estimating motion parameters of the field-of-view,

**characterised in that** the gradient-echo imaging protocol has been amended by inserting additional gradient blips (24) after at least some of the RF excitations (22), such that at least one additional gradient echo (25) is generated, allowing the acquisition of at least one additional k-space (4) line during one echo time.

2. The method of claim 1,
wherein the gradient-echo imaging protocol has been amended by inserting additional gradient blips (24) between at least some of the RF excitations (22) and the corresponding gradient echoes (30).

3. The method of claim 1 or 2, wherein the additional k-space lines (4) are acquired from a set of 2 to 16, preferably 4 to 8, k-space positions within a central region (16) of k-space, wherein each set of 2 to 16, preferably 4 to 8, additional k-space lines is used to estimate one motion state of the field-of-view, preferably a rigid-motion state.

4. The method of any one of the preceding claims, wherein the gradient-echo imaging protocol (21) is a steady-state imaging protocol, and/or a susceptibility-weighted imaging protocol.

5. The method of any one of the preceding claims, wherein the susceptibility-weighted gradient-echo imaging protocol (21) is a three-dimensional imaging protocol using a linear sampling order, and wherein one set of 2 to 16, preferably 4 to 8, additional k-space lines is acquired during each iteration of an inner phase-encoding loop (35) of the imaging protocol.

6. The method of any one of the preceding claims, wherein at least one additional k-space line (4, 25) is acquired during each echo time.

7. The method of any one of the preceding claims, wherein additional k-space lines (25) are used to estimate pose-dependent BO-field variations.

8. The method of any one of the preceding claims, wherein some of the additional k-space lines (4) are used to acquire a low-resolution image dataset of the field-of-view.

9. The method of any one of the preceding claims, wherein the imaging protocol (21) includes flow compensation gradients (28) inserted between the acquisitions of the additional k-space lines (25) and the imaging k-space lines (30), in order to reduce flow artefacts in the magnetic resonance image dataset.

10. The method of any one of the preceding claims, wherein the echo time (TE2) of the susceptibility-weighted gradient-echo imaging protocol is 10ms to 100ms, preferably 15ms to 50ms, more preferred 18ms to 25ms.

11. A method for generating a motion-corrected magnetic resonance image dataset of an object, comprising:

receiving k-space data (14) acquired using the acquisition method according to any one of claims 1 to 10, receiving a low-resolution scout image dataset (52) and sets of additional k-space lines (4) acquired using the acquisition method according to any one of claims 1 to 10;
in a first step (54), estimating motion parameters ($\theta_i$) for each set of additional k-space lines (4) by minimizing the data consistency error between the additional k-space lines (4) and a forward model using the low-resolution scout scan (52) as an estimate for the image dataset, wherein the forward model is described by an encoding matrix including the motion parameters ($\theta_i$) to be estimated, Fourier encoding (F), and optionally subsampling (M) and/or coil sensitivities (C) of a multi-channel coil array (7.1, 7.2); and
in a second step (56), estimating the motion-corrected image dataset (x) by minimizing the data consistency error between the k-space data (14) acquired in the imaging protocol and a forward model described by an encoding matrix, wherein the encoding matrix includes the motion parameters ($\theta_i$) estimated in the first step for each set of additional k-space lines (4), Fourier encoding (F), and optionally subsampling (M) and/or coil sensitivities (C) of a multi-channel coil array (7.1, 7.2) .

12. A magnetic resonance imaging apparatus (1) configured to execute the method of one of the preceding claims, comprising:

a radio frequency controller (7') configured to drive an RF-coil (7) comprising a multi-channel coil array (7.1, 7.2),
a gradient controller (5') configured to control gradient coils (5),
a control unit (13) configured to control the radio frequency controller (7') and the gradient controller (5') to execute the imaging protocol.

13. A computer program including program code, which causes a magnetic resonance imaging apparatus (1) to execute the method according to any one of claims 1 to 10.

## FIG 1

FIG 2

FIG 3

FIG 4

# FIG 5

$$[\hat{\chi}] = \mathrm{argmin}_\chi \, \| E_{\hat{\theta}}\chi - s \|_2^2$$

$$[\hat{\theta}] = \mathrm{argmin}_\theta \, \| E_\theta \hat{\chi} - s \|_2^2$$

# FIG 6

52

$$[\hat{\theta}_i] = \mathrm{argmin}_{\theta i} \, \| E_{\theta i}\hat{\chi} - s_i \|_2^2 \qquad 54$$

$$[\hat{\chi}] = \mathrm{argmin}_\chi \, \| E_{\hat{\theta}}\chi - s \|_2^2 \qquad 56$$

## EUROPEAN SEARCH REPORT

Application Number

EP 24 16 3711

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIU JIAEN ET AL: "Reducing motion sensitivity in 3D high-resolution T2*-weighted MRI by navigator-based motion and nonlinear magnetic field correction", NEUROIMAGE, ELSEVIER, AMSTERDAM, NL, vol. 206, 2 November 2019 (2019-11-02), XP086008187, ISSN: 1053-8119, DOI: 10.1016/J.NEUROIMAGE.2019.116332 [retrieved on 2019-11-02] * Sections 1 and 2 * * figure 1 * | 1-13 | INV. G01R33/565 |
| X | US 2022/065971 A1 (POLAK DANIEL [DE] ET AL) 3 March 2022 (2022-03-03) | 1-6,8-13 | |
| Y | * paragraph [0002] - paragraph [0047] * * figure 4 * | 7 | |
| X | EP 4 246 170 A1 (SIEMENS HEALTHCARE GMBH [DE]; MASSACHUSETTS GEN HOSPITAL [US]) 20 September 2023 (2023-09-20) | 1-6,8-13 | |
| Y | * paragraph [0008] - paragraph [0058] * * figures 2-5 * | 7 | TECHNICAL FIELDS SEARCHED (IPC) |
| Y | BRACKENIER YANNICK ET AL: "Data-driven motion-corrected brain MRI incorporating pose-dependent B0 fields", MAGNETIC RESONANCE IN MEDICINE, vol. 88, no. 2, 8 May 2022 (2022-05-08), pages 817-831, XP093191014, US ISSN: 0740-3194, DOI: 10.1002/mrm.29255 * Sections 1 and 2 * | 7 | G01R |

The present search report has been drawn up for all claims

| Place of search | Date of completion of the search | Examiner |
|---|---|---|
| Munich | 30 July 2024 | Streif, Jörg Ulrich |

EPO FORM 1503 03.82 (P04C01)

## ANNEX TO THE EUROPEAN SEARCH REPORT
## ON EUROPEAN PATENT APPLICATION NO.

EP 24 16 3711

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2024

| Patent document cited in search report | Publication date | Patent family member(s) | Publication date |
|---|---|---|---|
| US 2022065971 A1 | 03-03-2022 | NONE | |
| EP 4246170 A1 | 20-09-2023 | EP 4246170 A1 | 20-09-2023 |
| | | US 2023293039 A1 | 21-09-2023 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. HAMILTON** ; **D. FRANSON** ; **N. SEIBERLICH**. Recent Advances in Parallel Imaging for MRI,. *Prog. Nucl. Magn. Reson. Spectrosc.*, 2017, vol. 101, 71-95
- **L. CORDERO-GRANDE** ; **E. J. HUGHES** ; **J. HUTTER** ; **A. N. PRICE** ; **J. V HAJNAL**. Three-dimensional motion corrected sensitivity encoding reconstruction for multi-shot multi-slice MRI: Application to neonatal brain imaging. *Magn. Reson. Med*, 2018, vol. 79 (3), 1365-1376
- **L. CORDERO-GRANDE** ; **R. TEIXEIRA** ; **E. HUGHES** ; **J. HUTTER** ; **A. PRICE** ; **HAJNAL**. Sensitivity Encoding for Aligned Multishot Magnetic Resonance Reconstruction,. *IEEE Trans. Comput. Imaging*, 2016, vol. 2 (3), 266-280
- **M. W. HASKELL** ; **S. F. CAULEY** ; **L. L. WALD**. TArgeted Motion Estimation and Reduction (TAMER): Data consistency based motion mitigation for MRI using a reduced model joint optimization,. *IEEE Trans. Med. Imaging*, 2018, vol. 37 (5), 1253-1265
- Scout accelerated motion estimation and reduction (SAMER). *Magn. Reson. Med*, 2022, vol. 87, 163-178, https://doi.org/10.1002/mrm.28971
- **D. POLAK** ; **J. HOSSBACH** ; **D. N. SPLITTHOFF et al.** Motion guidance lines for robust data consistency-based retrospective motion correction in 2D and 3D MRI. *Magn. Reson. Med*, 2023, 1-14
- **D. POLAK et al.** *in Magn. Reson. Med.*, 2022, vol. 87, 163-178
- *Magn. Reson. Med.*, 2023, 1-14
- *Magn. Reson. Med.*, 2022, 163-178
- **Y. BRACKENIER**. Data-driven motion-corrected brain MRI incorporating pose-dependent B0-fields. *Magn. Reson. Med.*, 2022, vol. 88 (2), 817-831
- **K. P. PRUESSMANN** ; **M. WEIGER** ; **M. B. SCHEIDEGGER** ; **P. BOESIGER**. SENSE: sensitivity encoding for fast MRI. *Magn. Reson. Med.*, 1999, vol. 42 (5), 952-962